Europäisches Patentamt

European Patent Office

Office européen des brevets

(11) Publication number: **0 209 702**
**A1**

# EUROPEAN PATENT APPLICATION

(21) Application number: **86107846.7**

(22) Date of filing: **09.06.86**

(51) Int. Cl.⁴: **C 12 Q 1/68**
**G 01 N 33/543, G 01 N 33/569**

(30) Priority: **21.06.85 US 747230**

(43) Date of publication of application:
**28.01.87 Bulletin 87/5**

(84) Designated Contracting States:
**CH DE FR GB IT LI NL SE**

(71) Applicant: **MILES LABORATORIES, INC.**
**1127 Myrtle Street**
**Elkhart Indiana 46514(US)**

(72) Inventor: **Carrico, Robert J.**
**54256 Silver Street**
**Elkhart Indiana 46514(US)**

(74) Representative: **Adrian, Albert, Dr. et al,**
**c/o BAYER AG Konzernverwaltung RP Patentabteilung**
**D-5090 Leverkusen 1, Bayerwerk(DE)**

(54) Solid-phase hybridization assay using anti-hybrid antibodies.

(57) A nucleic acid hybridization method for detecting a part-
icular polynucleotide sequence present in a virus or cell contained in a test sample such as a biological fluid. The test
sample is treated to release nucleic acids from the virus or cell
of interest in single stranded form and to immobilize such
nucleic acids on a solid substrate such as a filter membrane.
Release of nucleic acids can be accomplished with alkali, lytic
enzymes, detergents, and the like. The immobilized nucleic
acids are contacted with a probe to form DNA·RNA or
RNA·RNA hybrids which are then detectable by binding of an
anti-hybrid antibody reagent, preferably labeled. The method
does not require the use of labeled probe and provides quan-
titative results due to the efficiency and stability of the im-
mobilization of sample nucleic acids.

## SOLID-PHASE HYBRIDIZATION ASSAY
## USING ANTI-HYBRID ANTIBODIES

### BACKGROUND OF THE INVENTION

This invention relates to nucleic acid hybridization assay methods for detecting specific polynucleotide sequences. Such methods find application in the fields of human and veterinary medicine, agriculture, and food science, among others. In particular, the technique can be used to detect and identify etiological agents such as bacteria and viruses, to screen bacteria for antibiotic resistance, and to detect cancerous cells.

The state-of-the-art nucleic acid hybridization assay techniques involve chemical modification of either the probe nucleic acid or sample nucleic acids for the purpose of labeling and detection. The necessity of chemically modifying nucleic acids severely limits the practical use of the technique since it requires the large-scale preparation of labeled probes involving complicated and expensive synthetic and purification procedures or the *in situ* synthesis of labeled sample nucleic acids by the analyst. In particular, the resulting labeled polynucleotide must retain the ability to hybridize efficiently with its complementary sample or probe

MS-1401

sequence. Such a requirement severely limits the availability of useful synthetic approaches to label modification of polynucleotides intended for use in hybridization assays.

The early hybridization techniques involved the use of radioactive labels such as $^3$H, $^{32}$P, and $^{125}$I. Labeled probes are synthesized enzymatically from radiolabeled nucleotides and a polynucleotide by such techniques as nick translation, end labeling, second strand synthesis, reverse transcription, and transcription. Thus, an additional requirement of such enzymatic methods is that the modified or labeled nucleotides must serve as effective substrates for the polymerase enzymes involved in the assembly of the labeled polynucleotide. Direct chemical modification of the polynucleotide is also possible, however, such methods are generally quite inefficient in incorporating labels into the polynucleotide and can affect the ability of the polynucleotide to undergo hybridization.

Because of the handling and storage disadvantages of radiolabeled materials, there has been considerable continuing efforts to develop useful nonradioisotopic labeling approaches. Such labels have included light emitting molecules such as fluorescers and chemiluminescers, and ligand molecules which are capable of being specifically bound by counterpart binders which are in turn labeled with detectable chemical groups such as fluorescers and enzymes. Examples of ligand labels are haptens, which are specifically bound by antibodies, and other small molecules for which

MS-1401

specific binding proteins exist, e.g., biotin which is bound by avidin.

British Pat. No. 2,019,408 describes polynucleotide probes which are labeled with biotin through cytochrome C linking groups and which are then detectable by enzyme-labeled avidin. An alternative approach to labeling probes with low molecular weight ligands such as biotin is described in European Pat. Appln. 63,879. In this technique, a 5-allylamine-deoxyuridine triphosphate (dUTP) derivative is condensed with the desired ligand label and the thus modified nucleotide is incorporated by standard enzymatic methods into the desired probe.

Techniques for detecting directly the polynucleotide duplex formed as the product of hybridization between the sample and probe polynucleotides, and thereby dispensing with the chemical labeling of one or the other polynucleotide, have been attempted. Antibodies which will selectively bind double stranded DNA·DNA hybrids over single stranded DNA have not been successfully produced [Parker and Halloran, "Nucleic Acids in Immunology", ed. Plescia and Braun, Springer-Verlag, NY (1969) pp. 18 et seq]. Some success has been achieved in generating antibodies that will bind DNA·RNA mixed hybrids and have low affinity for the single stranded polynucleotides [Rudkin and Stollar, Nature 265:472(1977); Stuart et al, PNAS(USA)78:3751(1981); Reddy and Sofer, Biochem. Biophys. Res. Commun. 103:959(1981); and Nakazato, Biochem. 19:2835(1980)].

MS-1401

The methods described in the literature involving the use of antibodies to DNA·RNA hybrids have all been restricted to a technique commonly referred to as *in situ* hybridization. In such technique, whole cells or virus particles from a test sample, usually a tissue section, are fixed onto microscope slides and the DNA in the nucleus is exposed by treatment with appropriate reagents such as ethanol, acetic acid, and enzymes. The exposed DNA is then hybridized with an RNA probe and the hybrids detected by fluorescence microscopy using fluorescently-labeled antibody to DNA·RNA. The cellular or viral DNA becomes in a sense immobilized on the microscope slide by being noncovalently adsorbed to the various cellular or viral elements left fixed to the slide. It is well-accepted in the art that the *in situ* hybridization technique is time consuming and unreliable and is only useful when the assay can be performed upon single cells (U.S. Pat. No. 4,483,920). Because of the variability and inefficiency in the immobilization and exposure of the sample DNA from the cell or virus inherent in such a process, its use has been restricted to qualitative analysis.

In other typical hybridization assays, a small amount of the labeled probe binds nonspecifically to the solid support used to immobilize the sample nucleic acids. This non-specifically bound probe gives a background signal which often sets the lower limit for detection of hybridized probe. The usual way to maintain the background signal at an acceptable level is to keep the probe concentration low during the hybridization step. However, this

MS-1401

tactic also slows the hybridization rate and extends the assay time.

Accordingly, there is a need to develop hybridization techniques which do not require labeling of the probe or sample nucleic acids and which provide an efficient and reproducible immobilization step. Further, such techniques should allow the use of a variety of labels, particularly of the nonradioisotopic type. Also, there is a need for techniques which permit the use of large excesses of the probe for faster hybridization kinetics. These and other features are objects of the present invention.

The use of antibodies to DNA'RNA or RNA'RNA hybrids in other types of nucleic acid hybridization assays is described in European Patent Application No. 146 039 entitled "Hybridization Assay with Immobilization of Hybrids by Anti-Hybrid Binding" and European Patent Application No. 163 220 entitled "Nucleic Acid Hybridization Assay Employing Detectable Anti-hybrid Antibodies"

## SUMMARY OF THE INVENTION

A nucleic acid hybridization method is provided by the present invention for determining particular polynucleotide sequences in a virus or cell contained in a test sample, usually a biological fluid. The test sample is treated to release a significant detectable amount of nucleic acid from the cell or virus of interest in single stranded form and to immobilize such single

MS-1401

stranded sample nucleic acids on a solid substrate. The immobilized nucleic acids are contacted with a soluble polynucleotide probe having a substantially complementary base sequence to the sequence to be detected. When the sequence to be detected is RNA, the probe is substantially composed of DNA or RNA, but when such sequence is DNA, the probe will be substantially composed of RNA. Hybridization between the probe and sequence of interest is allowed to proceed under favorable conditions. The resulting DNA·RNA or RNA·RNA hybrids are detected by adding an antibody reagent selective for binding such hybrids and then determining the antibody reagent that becomes bound to such hybrids by conventional methods, such as the use of a labeled form of the antibody reagent.

A principal feature of the present method is that the sample nucleic acids are released and separated from the major cellular or viral elements in the sample. In the process, the sample nucleic acids of interest if present as double stranded DNA will be denatured to single stranded form. The single stranded nucleic acids are then immobilized directly onto a suitable solid substrate. The overall immobilization process is thus independent of fixation of the sample nucleic acid to cellular or viral structural elements as is seen in the prior art *in situ* techniques. By appropriate selection of immobilizing substrates, an efficient and stable assay element can be achieved, enabling reproducible and quantitative analyses to be carried out.

MS-1401

## DESCRIPTION OF THE PREFERRED EMBODIMENTS

### *Sample Treatment*

A variety of materials and steps can be used in accomplishing the immobilization step of the present invention. One can first treat the test sample, whether a biological fluid or tissue sample, to produce an aqueous solution or suspension of nucleic acids released from the cells or viruses in the sample, and then, with or without first separating out the resulting cellular or viral debris, contact the solution or suspension with the desired immobilization substrate. Alternatively, one can collect or fix the sample onto a suitable substrate and then treating the deposited cells or viruses to release their nucleic acids for immobilization directly on the substrate, followed preferably by a wash to remove cellular or viral debris.

The release of a significant detectable amount of the nucleic acids in viruses or cells in the test sample can be accomplished in several ways. Normally, this involves treatment with alkali, lytic enzymes, and/or detergents useful for this purpose. Alkaline conditions such as 0.1 to 1.0 molar sodium hydroxide have been used to release DNA from bacteria [see Mosely et al (1982) J. Inf. Dis. 145:863] and this method also denatures the DNA for immobilization and hybridization. Other methods expose the bacteria to lytic enzymes such as lysozyme or lysostaphin followed by a detergent

MS-1401

such as sodium dodecylsulfate or Triton. Some bacteria can be lysed with detergent alone.

Blood cells, such as lymphocytes can be lysed by exposure to detergents such as sodium dodecylsulfate. In the case of tissue samples, it may be desirable to digest with a protease such as proteinase K or a protease in combination with a detergent and sodium perchlorate [Lizardi and Engelberg (1979) Anal. Biochem. 98:116]. In addition, a cell lysate (digest) can be extracted with phenol-chloroform, particularly if the cell debris interfere with the immobilization of the nucleic acids. Bresser et al (1983) DNA 2:243 describes a method for treating cell lysates with hot sodium iodide to solubilize nucleic acids and to facilitate immobilization on nitrocellulose.

Viral nucleic acids can usually be dissociated from coat proteins by exposure to detergents such as sodium dodecylsulfate [Virtanen et al (1983) Lancet 381; Teramato et al (1984) J. Clin. Micro 20:373]. Where desirable, digestion with a proteolytic enzyme and extraction with phenol can also be employed [Wichendom et al (1985) Lancet 65]. The nucleic acids are usually denatured with alkali or high temperature to facilitate immobilization and hybridization. High temperature is preferable for denaturation of double stranded RNA because it is destroyed by alkali.

*Immobilization*

As mentioned above, the immobilization step can actually be accomplished simultaneously with or subsequent to the release, and optional

MS-1401

denaturation, treatment. Essentially any available means can be used to immobilize the single stranded sample nucleic acids. Normally one will select from adsorption methods, covalent methods, and dual hybridization protocols.

The solid substrate can take on a variety of shapes and compositions, including microparticles, beads, porous and impermeable strips and membranes, the interior surface of reaction vessels such as test tubes and microtiter plates, and the like. Means for attaching the sample nucleic acids to a selected solid support will be a matter of routine skill to the worker in the field.

One method for adsorbing the sample nucleic acids onto nitrocellulose membranes involves saturating a solution of the nucleic acids with sodium iodide and spotting or filtering aliquots onto the membrane [Bresser et al (1983) DNA 2:243]. The sodium iodide facilitates denaturation of the nucleic acids and enhances adsorption onto the membrane. Alternatively, the nucleic acids can be treated with glyoxal, usually at concentrations around 1 molar, and then adsorbed onto the membrane. This method is particularly advantageous for RNA. The nucleic acids are fixed by baking at around 80°C under vacuum for a period in the range of 2-4 hours. [Thomas, P.S., (1983) Meth. in Enzymol. 100:255].

Covalent immobilization of nucleic acids can also be accomplished. A wide variety of support materials and coupling techniques can be employed. For example, the nucleic acids can be coupled to phosphocellulose through phosphate groups activated by carbodiimide or carbonyldiimidazole [Bautz,

MS-1401

E.K.F., and Hall, B.D., (1962) Proc. Nat'l. Acad. Sci. USA 48:400-408; Shih, T.Y., and Martin, M.A., (1974) Biochem. 13:3411-3418]. Also, diazo groups on m-diazobenzoyloxymethyl cellulose can react with guanine and thymidine residues of the polynucleotide [Noyes, B.E., and Stark, G.R., (1975) Cell 5:301-310; Reiser, J., et al, (1978) Biochem. Biophys, Res. Commun. 85:1104-1112]. Polysaccharide supports can also be used with coupling through phosphodiester links formed between the terminal phosphate of the polynucleotide and the support hydroxyls by water soluble carbodiimide activation [Richwood, D., (1972) Biochim. Biophys. Acta 269:47-50; Gilham, P.T., (1968) Biochem. 7:2809-2813], or by coupling nucleophilic sites on the polynucleotide with a cyanogen bromide activated support [Arndt-Jovin, D.J., et al, (1965) Eur. J. Biochem. 54:411-418; Linberg, U., and Eriksson, S., (1971) Eur. J. Biochem. 18:474-479]. Further, the 3'-hydroxyl terminus of RNA can be oxidized with periodate and coupled by Schiff base formation with supports bearing amine or hydrazide groups [Gilham, P.T., (1971) Method, Enzymol. 21:191-197; Hansske, H.D., et al, (1979) Method. Enzymol. 59:172-181]. Supports having nucleophilic sites can be reacted with cyanuric chloride and then with the polynucleotide [Hunger, H.D., et al, (1981) Biochim. Biophys. Acta 653:344-349].

Another approach to immobilizing the released single stranded sample nucleic acids involves the dual hybridization procedure as known in the art [Methods in Enzymol. 65:468(1968) and

MS-1401

Gene 21:77-86(1983)]. Following such a method, a second probe is used which has a base sequence complementary to a mutually exclusive portion of the sequence of interest relative to the first probe. The second probe is immobilized on the solid substrate using any of the previously described techniques or any other useful technique, and by hybridization with the sequence to be detected, thereby immobilizes same for detection by hybridization with the first probe.

A specialized modification of the dual hybridization method can be applied to measurements of messenger RNAs (mRNA) from eukaryotic cells which have poly(rA) tails on the 3'-OH ends. Polyadenylated RNAs in cell lysates can be immobilized by hybridization to immobilized poly(rT) or poly(rU). Then a specific DNA probe can be hybridized with the immobilized mRNAs and if a complementary sequence is present the DNA·RNA hybrid can be detected with labeled antibody to DNA·RNA.

## The Probe

The probe will comprise at least one single stranded base sequence substantially complementary to the sequence to be detected. However, such base sequence need not be a single continuous polynucleotide segment, but can be comprised of two or more individual segments interrupted by noncomplementary sequences. These nonhybridizable sequences can be linear, or they can be self-complementary and form hairpin loops. In addition, the complementary region of the probe can

MS-1401

be flanked at the 3'- and 5'-termini by nonhybridizable sequences, such as those comprising the DNA or RNA of a vector into which the complementary sequence had been inserted for propagation. In either instance, the probe as presented as an analytical reagent will exhibit detectable hybridization at one or more points with sample nucleic acids of interest. Linear or circular single stranded polynucleotides can be used as the probe element, with major or minor portions being duplexed with a complementary polynucleotide strand or strands, provided that the critical homologous segment or segments are in single stranded form and available for hybridization with sample DNA or RNA, and provided that the antibody reagent selected for use with the probe does not significantly crossreact with the double stranded regions in the probe (e.g., where the antibody reagent is specific for DNA·RNA hybrids and the probe comprises RNA·RNA double stranded regions, or vice versa). The complementary probe sequence can be of any convenient or desired length, ranging from as few as a dozen to as many as 10,000 bases, and including oligonucleotides having less than about 50 bases.

The RNA or DNA probe can be obtained in a variety of conventional manners. For example, in the case of RNA probes, RNA can be isolated as the natural products of cells, such as 5s, 16s and 23s ribosomal RNAs from bacteria or cellular transfer RNAs. It is also practial to isolate specific messenger RNAs from cells which specialize in

MS-1401

production of large amounts of a protein for which the messenger codes.

In vitro synthesis of RNA probes can be accomplished with a vector which contains the very active Salmonella typhimurium bacteriophage SP6 transcription promoter [Green et al (1983) Cell 32:681]. A vector with multiple restriction endonuclease sites adjacent to the promoter is available from Promega Biotec, Madison, WI, USA. A DNA probe is cloned into the vector which is then propagated in a bacterial host. Multiple RNA copies of the cloned DNA probe can be synthesized in vitro using DNA dependent RNA polymerase from bacteriophage SP6.

DNA probes can be prepared from a variety of sources. An entire bacterial genome can be immobilized for a hybridization assay designed to detect bacteria in a typically sterile sample. The assay would be capable of detecting abundant bacterial RNAs such as ribosimal RNAs and transfer RNAs. Alternatively, specific DNA sequences complementary to cellular RNAs can be cloned into well known plasmid or viral vectors and used as hybridization probes.

It should be understood that in using the expressions "RNA probe" and "DNA probe" herein, it is not implied that all nucleotides comprised in the probe be ribonucleotides or 2'-deoxyribonucleotides. The fundamental feature of an RNA or DNA probe for purposes of the present invention is that it be of such character to enable the stimulation of antibodies to DNA˙RNA or RNA˙RNA hybrids comprising an RNA or DNA probe which do not crossreact to an analytically significant degree

MS-1401

with the individual single strands forming such hybrids. Therefore, one or more of the 2'-positions on the nucleotides comprised in the probe can be chemically modified provided the antibody binding characteristics necessary for performance of the present assay are maintained to a substantial degree. Likewise, in addition or alternatively to such limited 2'-deoxy modification, a probe can have in general any other modification along its ribose phosphate backbone provided there is no substantial interference with the specificity of the antibody to the double stranded hybridization product compared to its individual single strands.

Where such modifications exist in an RNA or DNA probe, the immunogen used to raise the antibody reagent would preferably comprise one strand having substantially corresponding modifications and the other strand being substantially unmodified RNA or DNA, depending on whether sample RNA or DNA was intended to be detected. Preferably, the modified strand in the immunogen would be identical to the modified strand in an RNA or DNA probe. An example of an immunogen is the hybrid poly(2'-0-methyladenylic acid)·poly(2'-deoxythymidylic acid). Another would be poly(2'-0-ethylinosinic acid)·poly(ribocytidylic acid). The following are further examples of modified nucleotides which could be comprised in a modified probe: 2'-0-methylribonucleotide, 2'-0-ethylribonucleotide, 2'-azidodeoxyribonucleotide, 2'-chlorodeoxyribonucleotide, 2'-0-acetylribonucleotide, and the

0209702

phosphorothiolates or methylphosphonates of ribonucleotides or deoxyribonucleotides. Modified nucleotides can appear in probes as a result of introduction during enzymic synthesis of the probe from a template. For example, adenosine 5'-0-(1-thiotriphosphate) (ATPαS) and dATPαS are substrates for DNA dependent RNA polymerases and DNA polymerases, respectively. Alternatively, the chemical modification can be introduced after the probe has been prepared. For example, an RNA probe can be 2'-0-acetylated with acetic anhydride under mild conditions in an aqueous solvent [Steward, D. L. et al, (1972) Biochim. Biophys. Acta 262:227].

The critical property of an RNA or DNA probe for use herein is that antibodies raised against the probe duplexed with a complementary RNA or DNA strand, as desired, will discriminate in their binding properties between the duplexed form of the probe and single stranded nucleic acids. It is this property which enables detection of hybridized probe in the assay mixture without significant background binding to the unhybridized single stranded form of the probe or any nonspecifically bound single stranded sample nucleic acids. While as described above certain modifications along the ribonucleotide or deoxyribonucleotide strand can be tolerated without loss of antibody discrimination of the duplex from single strands, it will generally be preferable to employ RNA probes which are composed entirely of ribonucleotides when the sample polynucleotide is RNA or DNA. DNA probes can be used advantageously when the sample is RNA.

MS-1401

*Anti-Hybrid Antibody Reagent and Detection Schemes*

The antibody reagent of the present invention is principally characterized by its ability to bind the DNA·RNA or RNA·RNA hybrids formed between the probe and complementary sample nucleic acids to the significant exclusion of single stranded polynucleotides. The antibody reagent can consist of whole antibodies, antibody fragments, polyfunctional antibody aggregates, or in general any substance comprising one or more specific binding sites from an antibody for RNA·RNA or DNA·RNA, as the case may be. When in the form of whole antibody, it can belong to any of the classes and subclasses of known immunoglobulins, e.g., IgG, IgM, and so forth. Any fragment of any such antibody which retains specific binding affinity for the hybridized probe can also be employed, for instance, the fragments of IgG conventionally known as Fab, F(ab'), and F(ab')$_2$. In addition, aggregates, polymers, derivatives and conjugates of immunoglobulins or their fragments can be used where appropriate.

The immunoglobulin source for the antibody reagent can be obtained in any available manner such as conventional antiserum and monoclonal techniques. Antiserum can be obtained by well-established techniques involving immunization of an animal, such as a mouse, rabbit, guinea pig or goat, with an appropriate immunogen. The immunoglobulins can also be obtained by somatic cell hybridization techniques, such resulting in what are commonly referred to as monoclonal

MS-1401

antibodies, also involving the use of an appropriate immunogen.

Immunogens for stimulating antibodies specific for DNA·RNA hybrids can comprise homopolymeric or heteropolymeric polynucleotide duplexes. Among the possible homopolymer duplexes, particularly preferred is poly(rA)·poly(dT) [Kitagawa and Stollar (1982) Mol. Immunol. 19:413]. However, in general, heteropolymer duplexes will be preferably used and can be prepared in a variety of ways, including transcription of ϕX174 virion DNA with RNA polymerase [Nakazato (1980) Biochem. 19:2835]. ine selected RNA·DNA duplexes are adsorbed to a methylated protein, or otherwise linked to a conventional immunogenic carrier material, such as bovine serum albumin, and injected into the desired host animal [see also Stollar (1980) Meth. Enzymol. 70:70].

Antibodies to RNA·RNA duplexes can be raised against double stranded RNAs from viruses such as reovirus or Fiji disease virus which infects sugar cane, among others. Also, homopolymer duplexes such as poly(rI)·poly(rC) or poly(rA)·poly(rU), among others, can be used for immunization as above.

The binding of the antibody reagent to the hybridized probe duplex according to the present method can be detected by any convenient technique. Advantageously, the antibody reagent will itself be labeled with a detectable chemical group. Such detectable chemical group can be any material having a detectable physical or chemical property. Such materials have been well-developed in the field of immunoassays and in general most any label

MS-1401

useful in such methods can be applied to the present invention. Particularly useful are enzymatically active groups, such as enzymes (see Clin. Chem. (1976) 22:1243, U.S. Reissue Pat. No. 31,006 and UK Pat. 1,019,408), enzyme substrates (see U.S. Pat. No. 4,492,751, cofactors (see U.S. Pat. Nos. 4,230,797 and 4,238,565), and enzyme inhibitors (see U.S. Pat. No. 4,134,792); fluorescers (see Clin. Chem. (1979) 25:353); chromophores; luminescers such as chemiluminescers and bioluminescers (see U.S. Pat. No. 4,380,580); specifically bindable ligands such as biotin (see European Pat. Spec. 63,879) or a hapten (see PCT Publ. 83-2286); and radioisotopes such as $^{3}H$, $^{35}S$, $^{32}P$, $^{125}I$, and $^{14}C$. Such labels and labeling pairs are detected on the basis of their own physical properties (e.g., fluorescers, chromophores and radioisotopes) or their reactive or binding properties (e.g., enzymes, substrates, cofactors and inhibitors). For example, a cofactor-labeled antibody can be detected by adding the enzyme for which the label is a cofactor and a substrate for the enzyme. A hapten or ligand (e.g., biotin) labeled antibody can be detected by adding an antibody to the hapten or a protein (e.g., avidin) which binds the ligand, tagged with a detectable molecule. Such detectable molecule can be some molecule with a measurable physical property (e.g., fluorescence or absorbance) or a participant in an enzyme reaction (e.g., see above list). For example, one can use an enzyme which acts upon a substrate to generate a product with a measurable physical property. Examples of the latter include, but are not limited to, β-galactosidase, alkaline phosphatase and peroxidase. Other labeling schemes will be evident to one of ordinary skill in the art.

Alternatively, the antibody reagent can be detected based on a native property such as its own antigenicity. A labeled anti-(antibody) antibody will bind to the primary antibody reagent where the label for the second antibody is a conventional label as above. Further, antibody can be detected by complement fixation or the use of labeled protein A, as well as other techniques known in the art for detecting antibodies.

Where the antibody reagent is labeled, as is preferred, the labeling moiety and the antibody reagent are associated or linked to one another by direct chemical linkage such as involving covalent bonds, or by indirect linkage such as by incorporation of the label in a microcapsule or liposome which is in turn linked to the antibody. Labeling techniques are well-known in the art and any convenient method can be used in the present invention.

*Reaction Mixture*

The test sample to be assayed can be any medium of interest, and will usually be a liquid sample of medical, veterinary, environmental, nutritional, or industrial significance. Human and animal specimens and body fluids particularly can be assayed by the present method, including urine, blood (serum or plasma), milk, cerebrospinal fluid, sputum, fecal matter, lung aspirates, throat swabs, genital swabs and exudates, rectal swabs, and nasopharnygal aspirates.

As is known in the art, various hybridization conditions can be employed in the assay. Typically, hybridization will proceed at slightly elevated temperatures, e.g., between about 35 and 75°C and usually around 65°C, in a solution comprising buffer at pH between about 6 and 8 and with appropriate ionic strength (e.g., 2XSSC where 1XSSC = 0.15M sodium chloride and 0.015M sodium citrate, pH 7.0), protein such as bovine serum albumin, Ficoll (a trademark identifying a copolymer of sucrose and epichlorohydrin sold by Pharmacia Fine Chemicals, Piscataway, NJ, USA), polyvinylpyrrolidone, and a denatured foreign DNA such as from calf thymus or salmon sperm. The degree of complementarity between the sample and probe strands required for hybridization to occur depends on the stringency of the conditions as known in the art.

Normally, the temperature conditions selected for hybridization will be incompatible with the binding of antibody reagent to formed hybrids and detection of the label response. Accordingly, the antibody reagent binding step and label detection step will proceed after completion of the hybridization step. The reaction mixture will usually be brought to a temperature in the range of from about 3°C to about 40°C and the binding and detection steps then performed.

It might be found in a particular assay situation using an RNA probe that the probe is subject to partial degradation by alkaline hydrolysis of the phosphodiester bonds or by the

MS-1401

presence of ribonucleases. In the former case, hydrolysis can be controlled by avoiding exposure of the probe to a pH higher than about 10. Ribonucleases can be effectively inhibited by the presence of such substances as sodium dodecylsulfate, aurintricarboxylic acid, ribonucleoside vanadyl complexes, heparin, diethylpyrocarbonate, and proteinaceous inhibitors isolated from mammalian sources.

The present invention will now be illustrated, but is not intended to be limited, by the following examples.

## Example I

### Hybridization Assay for Detection of Bacteria in Urine

A. Preparation of methylated thyroglobulin

One hundred milligrams of bovine thyroglobulin (Sigma Chemical Co., St. Louis, MO, USA) is combined with 10 ml of anhydrous methanol and 400 $\mu$l of 2.55 M HCl in methanol. This mixture is stirred on a rotary mixer at room temperature for 5 days. The precipitate is collected by centrifugation and washed twice with methanol and twice with ethanol.

B. Preparation of antibody to DNA·RNA hybrid

A DNA·RNA hybrid is prepared by transcription of ϕX174 virion DNA with RNA polymerase as described by Nakazato [Biochem. 19:2835(1980)].

MS-1401

One hundred fifty (150) micrograms (µg) of the hybrid in 250 µL of 20 mM Tris-hydrochloride buffer, pH 7.4, 1 mM ethylenediaminetetraacetate (EDTA) is combined with 150 µg of methylated thyroglobulin in 250 µL water. A precipitate forms and is suspended in Tris-buffer. This mixture is emulsified with an equal volume of Freunds adjuvant. BALB/c mice are immunized subcutaneously with 10 µg of hybrid per injection. The immunizations are repeated on days 14, 21, 28 and 35 and test bleedings are taken on days 23 and 37. Serums are titered for antibodies by enzyme immunoassay using Immunolon II microtiter plates (Dynatech, Alexandria, VA) in which the wells are coated with DNA·RNA hybrid. The coating is accomplished by placing 100 µL of 0.015M sodium citrate, pH 6.8, 0.15M NaCl containing 5.0 µg DNA·RNA/mL in each well and allowing it to stand for 2 hours at room temperature. Then the wells are washed three times with 20 mM sodium phosphate buffer, pH 7.4, 0.15M NaCl, 5 mg bovine serum albumin/mL and 0.5% (v/v) Tween 20.

Spleens from mice with high titers to DNA·RNA and low titers to single stranded DNA are fused with SP 2/0-Ag 14 myeloma cells and the resulting hybridomas are screened for those producing antibodies specific for DNA·RNA [Stuart et al (1981) Proc. Natl. Acad. Sci. USA 78, 3751, Galfre and Milstein (1981) Meth. in Enzymol. 73, 1]. A particularly preferred hybridoma is that deposited with the American Type Culture Collection, Rockville, MO, USA, as ATCC HB 8730.

The cloned hybridomas are propagated in the peritoneal cavity of mice to generate a large

MS-1401

quantity of antibody.  The antibody is purified from the ascites fluid by high pressure liquid chromatography on an anion-exchange column, Bakerbond MAb$^{TM}$ (J. T. Baker Research Products, Glenn Ellyn, IL, USA).  The ascites fluid is dialyzed into 10 mM potassium phosphate buffer, pH 6.8, centrifuged to remove precipitate and passed through a 0.22 μm nitrocellulose filter.  One to two milliliters of ascites fluid is applied to a 10 x 250 mm anion-exchange column equilibrated with 10 mM potassium phosphate buffer, pH 6.84.  The chromatography is developed with a 60 min linear gradient from 10 mM potassium phosphate buffer, pH 6.85 to 85 mM potassium phosphate, pH 6.40 at a flow rate of 1.0 mL/min.  The absorbance of the effluent at 254 nm is monitored and the peak with the IgG is collected.

C.    Labeling of antibody to DNA·RNA with β-Galactosidase

The purified antibody to DNA·RNA is dialyzed against 0.1 M sodium acetate buffer, pH 4.2, overnight and any insoluble material that forms is removed by centrifugation.  A 3.3 mL aliquot containing 10.5 mg IgG is combined with 64 μL of pepsin (5 mg/mL) and incubated at 37°C for 16 hours.  The digestion is quenched by addition of 0.5 ml of 2.0 M Tris-base to give pH 8.0.

The digest is concentrated to about 0.5 ml on a pressure dialysis membrane and then chromatographed on a 1.4 x 57 cm column of Sephacryl-200 (Pharmacia) equilibrated with 10 mM sodium phosphate buffer, pH 7.0, 0.15 M NaCl.

MS-1401

Fractions of 2.5 mL are collected and the absorbances at 280 nm are measured. The first peak eluted, fractions 17 to 20, are pooled to yield 4.2 mg of (Fab')$_2$.

Eight milligrams of β-galactosidase from E. Coli (Sigma Chemical Co., St. Louis, MO, USA) in 100 mM sodium phosphate buffer, pH 7.0, 0.15 M NaCl is combined with 35 μL of 0.1 M dithiothreitol and allowed to stand at 25°C for 4.0 hours. The mixture is chromatographed on a 1 x 25 cm column of BioGel P-6DG (BioRad Laboratories, Richmond, CA, USA) equilibrated with the same buffer. One milliliter fractions are collected and the enzyme is recovered in the first peak with absorbance at 280 nm. The protein is concentrated to 2.5 ml and to yield 7.1 mg. This enzyme has 10.4 sulfhydryl groups as determined by the method of Ellman [Ellman (1959) Arch. Biochem. Biophys. 82:70].

A 18.2 mM solution of N,N'-o-phenylenedimaleimide (Aldrich Chemical Co., Milwaukee, WI, USA) is prepared in dimethylformamide and 121 μL is added to 2.1 ml of the reduced β-galactosidase (2.8 mg/mL). This mixture is allowed to react at room temperature for 1.0 hour and then it is chromatographed on the BioGel P-6DG column described above and the modified enzyme is recovered in the first eluted peak of material with absorbance at 280 nm.

Four milligrams (1.45 ml) of the (Fab')$_2$ preparation described above is combined with 143 μL of 1.0 M sodium phosphate buffer, pH 7.0, 200·μL of 100 mM dithiothreitol and 20 μL of 100 mM EDTA. The mixture is allowed to stand at room temperature for 3 hours and then is chromatographed on a 1 x 25

MS-1401

cm column of BioGel P-6DG equilibrated with 10 mM sodium phosphate buffer, pH 7.0, 0.15 M NaCl and 1.0 mM EDTA. The first eluted peak of material with absorbance at 280 nm is pooled and concentrated to 2.5 mL. Typically, the protein has an average of 2.5 sulfhydryl groups per Fab' as determined by the method of Ellman, supra.

Four milliliters of the activated β-galactosidase, 3.4 mg, is combined with 1.1 ml of the Fab', 1.45 mg, and allowed to react for 22 hours at 4°C.

Insoluble material which forms is removed by centrifugation and the supernatant is chromatographed on a 1.4 x 44 cm column of BioGel A1.5 m (BioRad Laboratories) equilibrated with 10 mM sodium phosphate buffer, pH 7.0, 0.15 M NaCl. Two milliliter fractions are collected and the absorbances at 280 nm are measured. Also, the β-galactosidase activity in each fraction is assayed using o-nitrophenyl-β-galactoside as substrate. Fractions 14 to 18 typically have strong absorbances at 280 nm and enzyme activity and they are pooled.

D.    Preparation of ribosomal RNA probe

A mixture of 16 and 23s ribosomal RNAs is isolated from E. coli by the method of Takanami (1967) Meth. Enzymol. 12A:491.

MS-1401

E.   Hybridization assay

A 0.45 µm pore size nitrocellulose membrane (Schleicher and Schull, Keene, NH, USA) is mounted in a multiwell Minifold apparatus (Schleicher and Schuell) and 1.0 ml aliquots of urines suspected of having bacteria are filtered through the membrane. Then the membrane is placed on filter paper saturated with 0.3 M NaOH for 3.0 hours at room temperature.  This alkali disrupts bacteria on the membrane, degrades any RNA present and denatures the DNA.  The membrane is placed on filter paper saturated with 0.2 M sodium phosphate buffer, pH 7.0, for 30 minutes to neutralize the alkali and then the membrane is dried at 80°C in vacuo for 2 hours.

Hybridization is conducted by placing the membrane in a plastic pouch containing 0.25 ml/cm$^2$ of a solution containing 4 parts by volume of formamide and 6 parts 33 mM sodium phosphate buffer, pH 7.7, 0.5 M NaCl, 3.3 mM EDTA, 0.1% (w/v) sodium dodecylsulfate, 0.5 mg bovine serum albumin/mL, 0.5 mg Ficoll/mL, 0.5 mg polyvinylpyrrolidone/mL, 0.2 mg salmon sperm DNA/mL and 100 ng of the purified E. coli ribosomal RNA/mL.  (Prior to use the salmon sperm DNA is incubated at 37°C for 16 hours in 0.3 M NaOH and then neutralized with 5M acetic acid.)

The plastic pouch is sealed and placed in a water bath at 55°C for 17 hours.  Then the membrane is rinsed twice for 5 minutes each in 20 mM sodium phosphate buffer, pH 7.4, 0.3 M NaCl, 0.2 mM EDTA and 1.0 mg sodium dodecylsulfate/mL.  Next it is

MS-1401

washed for 15 minutes in 1.0 mL/cm$^2$ of 50 mM sodium phosphate buffer, pH 7.6, containing 5 mM MgCl$_2$, 5.0 mg BSA/mL, 0.5% (v/v) Tween 20. The β-galactosidase anti-DNA·RNA conjugate is diluted into this sodium phosphate buffer solution at 200 ng/mL and spread uniformly over the membrane at 0.2 mL/cm$^2$. The wet membrane is allowed to stand at room temperature for 1 hour and care is taken to prevent drying.

Excess enzyme-antibody conjugate is washed from the membrane with three five minute washes each composed of 2.5 mL/cm$^2$ of the sodium phosphate buffer solution containing 0.5 M NaCl. Finally, enzyme-antibody conjugate bound to DNA·RNA hybrids on the membrane is detected by applying 0.2 mL/cm$^2$ of 50 mM sodium phosphate buffer, pH 7.6, 5 mM MgCl$_2$ and 1.0 mM 5-bromo-4-chloro-3-indolyl-β-D-galactopyranoside (Sigma). The substrate solution is allowed to stand in contact with the membrane at room temperature for 1 hour with care to prevent drying. Areas of the membrane which filtered urines with more than $4 \times 10^5$ bacteria/mL develop blue color which is distinguishable from background. The test is most sensitive for gram negative bacteria.

Example II


Hybridization Assay for Cytomegalovirus in Urine


A.    Preparation of an RNA probe for
      cytomegalovirus


A DNA fragment from the genome of cytomegalovirus is cloned into a vector containing the promoter for DNA dependent RNA polymerase from bacteriophage SP6 that infects Salmonella typhimurium LT2 cells.  The cloned sequence is transcribed by the polymerase to produce an RNA probe.

Cytomegalovirus DNA is digested with EcoRI restriction endonuclease and the fragments are cloned into the plasmid pACYC 184 [Tamashiro et àl (1982) J. Virology 42:547-557].  The plasmids are propagated in E. coli strain HB101 and the EcoRI e fragment defined in the Tamashiro reference is used for preparation of the probe.  The purified plasmid is digested with EcoRI restriction endonuclease and the insert is isolated by agarose gel electrophoresis [Maniatis et al (1982) "Molecular Cloning", Cold Spring Harbor Laboratory].  The cytomegalovirus EcoRI e fragment is cloned into the EcoRI site of the pSP65 vector available from Promega Biotec, Madison, WI, USA.

The pSP65 vector with the EcoRI e insert is propagated in E. coli JM103 cells at 37°C.  The cells are lysed and the cellular DNA is isolated by phenol/chloroform extractions.  The plasmid is separated from the chromosomal DNA by centrifugation in a cesium chloride-ethidium bromide gradient [Maniatis et al, supra].

The cesium chloride and ethidium bromide are separated from the plasmid by gel filtration on Sephadex G-50 (Pharmacia Fine Chemicals) in 10 mM Tris-hydrochloride buffer, pH 7.5, containing 50 mM NaCl and 1 mM EDTA. The effluent containing DNA is collected and the DNA is precipitated with cold ethanol. The precipitate is dissolved in 10 mM Tris-hydrochloride buffer, pH 7.5, containing 50 mM NaCl, 10 mM $MgCl_2$ and 1 mM dithiothreitol and digested for 1 hour at 37°C with 1 unit Hind III restriction endonuclease per microgram DNA. The mixture is extracted once with phenol/chloroform and the DNA is precipitated with cold ethanol. The precipitate is dissolved in 10 mM Tris-hydrochloride buffer, pH 7.4, to give 0.5 mg DNA/mL.

This pSP65 vector with the EcoRI e insert is transcribed with SP6 DNA dependent RNA polymerase starting at the promoter and ending at the site cut by Hind III restriction endonuclease. Most of the DNA in the transcribed region is the EcoRI e insert.

The transcription reaction mixture (500 µL) has the following composition: 50 µg of the Hind III digested DNA; 40 mM Tris-hydrochloride buffer, pH 7.5; 6 mM $MgCl_2$; 2 mM spermine; 0.5 mM ATP, CTP, UTP and GTP; 10 mM dithiothreitol; 500 units RNasin (Promega Biotec) and 50 units of SP6 RNA polymerase (Promega Biotec). The reaction is allowed to proceed for 1 hour at room temperature and then an additional 50 units of RNA polymerase is added and allowed to react for one hour.

MS-1401

DNA in the mixture is destroyed by digestion for 10 minutes at 37°C with 10 µg of RNase-free DNase. The reaction mixture is extracted with phenol/chloroform and chromatographed on Sephadex G-50 in 10 mM Tris-hydrochloride buffer, pH 7.4, 0.1 M NaCl. The RNA is collected and precipitated with cold ethanol. The precipitate is dissolved in 50 mM sodium acetate buffer, pH 6.0, containing 1 mM EDTA.

B.    Hybridization assay

Cytomegalovirus is isolated from urine by centrifugation and the viral DNA is denatured and immobilized on a nitrocellulose membrane. The DNA is hybridized with the RNA probe and DNA·RNA hybrids formed are detected with the β-galactosidase-anti-DNA·RNA conjugate.

Cells and cell debris are removed from urine by centrifugation in a Sorvall GLC-3 centrifuge at 3000 rpm for 5 minutes. Ten milliliters of supernatant is placed in a polyallomer ultracentrifuge tube and run at 25,000 rpm in a Beckman Ti50 rotor for 75 minutes. The supernatant is removed and the pellet is dissolved in 50 µL of 0.1 M NaOH and incubated at 37°C for 2 hours.

Twenty microliters of 0.5 M sodium phosphate buffer, pH 6.0, containing 10 mM EDTA is added and the mixture is spotted on Gene Screen Plus Microporous membrane (New England Nuclear Research Products, Boston, MA, USA). The membrane is allowed to air-dry to fix the DNA to the surface.

Hybridization is conducted as described in Example I above except that 0.1 µg of the

MS-1401

cytomegalovirus RNA probe is used per mL of hybridization solution and the hybridization temperature is 40°C. Immunodetection of DNA·RNA hybrids is carried out as in Example I. Urines infected with cytomegalovirus give a blue spot on the membrane and uninfected urines give a color equivalent to the background observed on areas of the membrane not exposed to urine sediments.

The above examples provide representative embodiments of the present invention. Obviously many other modifications can be made without departing from the inventive features of the present method.

Claims:

1. A method for determining a particular polynucleotide sequence present in a virus or cell contained in a test sample, wherein nucleic acids from the virus or cell are contacted with a polynucleotide probe comprising at least one single stranded base sequence which is substantially complementary to the base sequence to be determined, such contact being performed under conditions favorable to hybridization between the sequence to be determined and the complementary probe sequence, and wherein resulting hybrids are detected,

characterized in that the test sample is treated to release a significant detectable amount of nucleic acids from the virus or cell in single stranded form and to immobilize such sample nucleic acids on a solid support, the polynucleotide probe being (i) substantially composed of RNA when the sequence to be determined is DNA, or (ii) substantially composed of DNA or RNA when the sequence to be determined is RNA, and the hybridized probe is detected by adding an antibody reagent capable of binding to DNA·RNA or RNA·RNA duplexes formed between the sequence to be determined and the complementary probe sequence and determining the antibody reagent that becomes bound to such duplexes.

2. The method of Claim 1 wherein the test sample is treated to release and denature a significant detectable amount of nucleic acids from said virus or cell and the resulting single stranded nucleic acids are immobilized on a solid substrate.

3. The method of any of Claims 1 and 2 wherein the virus or cell is immobilized on the solid substrate and then treated to release and denature a significant detectable amount of

MS 1401

nucleic acids therefrom, the resulting single stranded nucleic acids becoming immobilized on such substrate.

4. The method of any of Claims 1 to 3 wherein the test sample is a biological fluid and the substrate is a filter through which the biological fluid sample is passed to immobilize such virus or cell.

5. The method of any of Claims 1 to 4 wherein the single stranded sample nucleic acids are immobilized on the solid substrate by adsorption.

6. The method of any of Claims 1 to 5 wherein the single stranded sample nucleic acids are immobilized on the solid substrate by contact with a solid substrate to which is bound a polynucleotide having a base sequence complementary to a mutually exclusive portion of the nucleic acid containing the sequence to be determined and to which the probe hybridizes.

7. The method of any of Claims 1 to 6 wherein the antibody reagent is labeled with a detectable chemical group, preferably an enzymatically active group such as an enzyme, a fluorescer, a chromophore, a luminescer, a specifically bindable ligand, or a radioisotope.

8. The method of Claim 7 wherein the labeled antibody reagent which becomes bound to said duplexes is separated from that which does not become so bound and wherein the detectable chemical group is measured in one of the separated fractions.

9. The method of any of Claims 1 to 8 wherein the particular polynucleotide sequence to be determined is RNA or DNA and said probe is substantially composed of RNA.

MS 1401

10. The method of any of Claims 1 to 8 wherein the particular polynucleotide sequence to be determined is RNA and said probe is substantially composed of DNA.

MS 1401

## EUROPEAN SEARCH REPORT

European Patent Office

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| D,P X | EP-A-0 163 220 (MILES LABORATORIES INC.) * Whole document * | 1-10 | C 12 Q 1/68 G 01 N 33/543 G 01 N 33/569 |
| X | EP-A-0 144 913 (MILES LABORATORIES INC.) * Whole document * | 1-10 | |
| X | EP-A-0 133 671 (MILES LABORATORIES INC.) * Abstract; pages 21-26; claims 1,3,4,10 * | 1-10 | |
| X | EP-A-0 135 159 (CETUS CORP.) * Whole document * | 1-10 | |
| A | EP-A-0 145 356 (NATIONAL RESEARCH DEVELOPMENT) * Abstract; claims 1-9; figure 1/1 * | 1,6 | TECHNICAL FIELDS SEARCHED (Int Cl 4) C 12 Q |
| A | EP-A-0 079 139 (ORION CORP. LTD.) * Abstract; claims 1-12 * | 1,6 | |
| A | EP-A-0 133 288 (MILES LABORATORIES INC.) * Pages 3,4,12-15; examples 1-3; claims 1-10 * | 1-5 | |

---  -/-

The present search report has been drawn up for all claims

| Place of search | Date of completion of the search | Examiner |
|---|---|---|
| THE HAGUE | 24-10-1986 | OSBORNE H.H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO Form 1503 03 82

European Patent Office

**EUROPEAN SEARCH REPORT**

Application number

EP 86 10 7846

| | DOCUMENTS CONSIDERED TO BE RELEVANT | | Page 2 |
|---|---|---|---|
| Category | Citation of document with indication, where appropriate, of relevant passages | Relevant to claim | CLASSIFICATION OF THE APPLICATION (Int. Cl.4) |
| A | GENE, vol. 21, 1983, pages 77-85, Elsevier Biomedical Press; M. RANKI et al.: "Sandwich hybridization as a convenient method for the detection of nucleic acids in crude samples" * Whole document * | 1,6 | |
| D,A | US-A-4 483 920 (D. GILLESPIE et al.) | | |

TECHNICAL FIELDS SEARCHED (Int. Cl.4)

The present search report has been drawn up for all claims

| Place of search | Date of completion of the search | Examiner |
|---|---|---|
| THE HAGUE | 24-10-1986 | OSBORNE H.H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82